# EUROPÄISCHE PATENTSCHRIFT

(11) **EP 3 588 092 B1**
(45) Veröffentlichungstag und Bekanntmachung des Hinweises auf die Patenterteilung: **24.08.2022**
(21) Anmeldenummer: 18179915.6
(22) Anmeldetag: 26.06.2018
(51) Int. Cl.: G01N 33/84

(54) **MARKIERUNG VON ALUMOSILIKATEN**
MARKING ALUMINOSILICATES
MARQUAGE DES ALUMINOSILICATES

(43) Veröffentlichungstag der Anmeldung: 01.01.2020
(73) Patentinhaber: Glock Health, Science and Research GmbH, 2232 Deutsch Wagram (AT)
(72) Erfinder: Glock, Gaston, 9220 Velden (AT)
(74) Vertreter: Patentanwälte Barger, Piso & Partner

(56) Entgegenhaltungen:
- J. J. POWELL: "Analysis of aluminosilicate particles in biological matrices using histochemistry and X-ray microanalysis", THE ANALYST, Bd. 127, Nr. 6, 16. Mai 2002 (2002-05-16), Seiten 842-846, XP055511195, ISSN: 0003-2654, DOI: 10.1039/b110761p
- D. J. HYDES ET AL: "Fluorimetric method for the determination of low concentrations of dissolved aluminium in natural waters", THE ANALYST, Bd. 101, Nr. 1209, 1. Januar 1976 (1976-01-01), Seiten 922-931, XP055511235, ISSN: 0003-2654, DOI: 10.1039/an9760100922
- I. MILE ET AL: "Al adjuvants can be tracked in viable cells by lumogallion staining", JOURNAL OF IMMUNOLOGICAL METHODS, Bd. 422, 2015, Seiten 87-94, XP029239940, ISSN: 0022-1759, DOI: 10.1016/J.JIM.2015.04.008

## Beschreibung

Die Erfindung betrifft eine spezifische Markierung von Alumosilikaten, insbesondere den spezifischen Nachweis und die Markierung von Zeolith-Partikeln in unterschiedlichen Matrices in Übereinstimmung mit dem einleitenden Teil des Anspruches 1 und der weiter unten erläuterten Veröffentlichung J.J. Powell, 2002.

Der eindeutige Nachweis von Alumosilikaten in biologischen Proben ist aufgrund ihres vielfältigen Einsatzes von entscheidender Bedeutung. Alumosilikate werden beispielsweise im Agrarbereich als natürlicher Dünger in den Boden eingebracht. In der Viehzucht und der Mast dienen Alumosilikate zur Gesunderhaltung und Förderung der Gewichtszunahme der Tiere. Zur Anwendung im bzw. am Menschen gelangen Alumosilikate ebenso und befinden sich in ursprünglicher oder modifizierter Form in Verkehr (z.B. zusammengefasst von Mumpton, 1999). Trotz der breiten Verwendung von Alumosilikaten ist deren Nachweis aufgrund von hohen technischen Erfordernissen höchst anspruchsvoll und schwierig.

Generell können Alumosilikat-Verbindungen direkt über Röntgendiffraktion (XRD, spezifisch - jedoch relativ unempfindlich) oder indirekt über Elektronenmikroskopie (unspezifisch) nachgewiesen werden.

Die Verwendung Aluminium-spezifischer Färbemethoden in der biologischen Forschung umfasst die Verwendung von Chromophoren wie Hämatoxylin, Eriochrom Zyanin R, Aluminon und azidem Solochrom Azurin. Da diese aber relativ unspezifisch sind und nur geringe räumliche Auflösung zeigen, konnten sie nicht für Studien der zellulären Verteilung von Aluminium-Ionen verwendet werden (Eticha et al., 2005). Zu diesem Zwecke wurden die Fluorophore Morin (2',3,4',5,7-Pentahydroxyflavon) und Lumogallion (5-Chloro-3-(2,4-dihydroxyphenylazo)-2-hydroxybenzensulfonsäure) eingesetzt, die beide für den Nachweis von Aluminium geeignet sind (Illes et al., 2006), wobei sich Morin zumindest teilweise weniger spezifisch und sensitiv im Vergleich zu Lumogallion verhielt (Kataoka et al., 1997). Ein Beispiel dafür ist, dass Alumosilikatpartikel eingebettet in Gelatine oder in Darmgewebe sich nicht über Morin nachweisen ließen (Powell, 2002).

Die histochemischen Färbungen Solochrom Azurin, Solochrom Zyanin und Aluminon wurden von Powell für die direkte Färbung von Alumosilikatpartikeln getestet und erwiesen sich als ungeeignet dafür (Powell, 2002).

Die Lokalisation von Aluminium-Ionen-Verbindungen umfasst - ganz im Gegensatz zu in Alumosilikatpartikeln gebundenem Aluminium - durch die Verwendung von Lumogallion in diversen biologischen Fragestellungen während der letzten Jahrzehnte ein äußerst breites Themen-Spektrum:
1. Wasser:
   Eines der ersten Anwendungsgebiete von Lumogallion war die Identifikation von gelöstem Aluminium in Wasser-Proben (z.B.: Hydes und Liss, 1976; Caschetto und Wollast, 1979; Jonasson, 1980; He et al., 1997; Ren et al., 2001), die von Nishikawa und Kollegen erstmals veröffentlich wurde (Nishikawa et al., 1967 und 1968; Shigematsu et al., 1970).
   He et al., 1997 testeten die Spezifität von Lumogallion auf Ionen (bei einer Konzentration von 10 ppm pro Element), die in natürlichem Wasser vorkommen können: Fe³⁺, Ca²⁺, Mg²⁺, Cu²⁺ und Zn²⁺ und bestätigten die Selektivität der Reaktion von Lumogallion mit Al³⁺ (He et al., 1997).
   Mirza und Kollegen betteten verschiedene Formen von Metall-Ionen (Al(III), Ca(II), Cu(II), Fe(III), Mg(II), Zn(II)) in Agarose ein und stellten daraus Schnitte her, welche sie im Fluoreszenzmikroskop nach Lumogallion-Färbung analysierten. Sie fanden nur eine Fluoreszenz-Reaktion mit Lumogallion bei Verwendung der Schnitte von dreiwertigem Aluminium (Mirza et al., 2016).
   Sowohl T.R. Crompton als auch G. Sposito zitieren in ihren Büchern die Veröffentlichung von Hydes und Liss aus dem Jahre 1976 und schlussfolgern, dass "All forms of aluminum in filtered water may be detected except when the aluminum occurs in stable mineral structures, such as clay particles, small enough to pass through the filter." ["Alle Formen von Aluminium können im gefilterten Wasser detektiert werden, mit Ausnahme wenn das Aluminium in stabilen mineralischen Strukturen vorkommt, wie beispielsweise in Ton-Partikeln, welche klein genug sind um durch den Filter zu gelangen." Übersetzung des Anmelders.]
   (Crompton, 2015) und "Lumogallion does not react with suspended clay minerals, but the method does seem to determine small quantities of absorbed aluminum on kaolinite." ["Lumogallion reagiert nicht mit suspendierten Ton-Mineralien, aber es scheint, dass diese Methode kleine Mengen von an Kaolinit absorbiertem Aluminium bestimmen kann." Übersetzung des Anmelders.] (Sposito, 1995).
   In der im Jahre 2002 erschienen Arbeit von J.J. Powell wird die Unmöglichkeit einer Färbung von Aluminosilikaten mit histochemischen Färbemethoden (Solochrom-Azurin, Solochrom-Cyanin, Aluminon, Morin) im Gegensatz zu Aluminiumhydroxid und Aluminiumphosphat wie folgt begründet: "Both aluminium hydroxide and aluminium phosphate are insoluble at physiological pH and are well bound forms of the metal. However, sufficient aluminium ions are available for chelation at the surface of these species to give strong, positive reaction with the stains used here. In contrast, aluminosilicates, which are especially well bound forms of aluminium, clearly have insufficient available aluminium ions at their surfaces for detection by conventional histochemical staining and other methods were therefore sought." ["Sowohl Aluminiumhydroxid als auch Aluminiumphosphat sind bei physiologischem pH unlöslich und gut gebundene Formen des Metalls. Jedoch sind ausreichend Aluminium-Ionen für eine Chelatisierung auf der Oberfläche dieser Arten vorhanden, um eine starke, positive Reaktion mit den hier verwendeten Färbungen zu erhalten. Konträr dazu haben Alumosilikate, die besonders gut gebundene Formen von Aluminium sind, eindeutig nicht ausreichend verfügbare Aluminium-Ionen auf ihrer Oberfläche um diese durch konventionelle histochemische Färbungen detektieren zu können, daher wurden andere Methoden hierfür nötig." Übersetzung des Anmelders.] J.J. Powell schlussfolgert: "Thus in conclusion it was not feasible to demonstrate aluminium histochemically when it was bound to aluminosilicates." ["Daraus wird schlussgefolgert, dass es nicht möglich ist, Aluminium histochemisch nachzuweisen, wenn dieses an Alumosilikaten gebunden ist." Übersetzung des Anmelders.] (Powell, 2002).
2. Pflanzen:
   Mehr als 40 % der landwirtschaftlich nutzbaren Böden weltweit weisen einen sehr sauren pH-Wert (< 5) auf, wodurch die Aluminium-Verbindung Al³⁺ aus einem Lehm-enthaltenden Boden frei wird und direkt auf die Pflanzen wirkt, was oftmals zu drastischen Ernteausfällen führt (Sivaguru et al., 2013). Daher ist die Erforschung von Auswirkungen des Aluminiums auf Pflanzen weltweit von besonderem Interesse. Sivaguru et al. 2013 beschrieben einen Resistenzmechanismus einer Hirseart (*Sorghum bicolor*) gegen Aluminium, welchen sie in den Wurzeln der Pflanze mittels Lumogallion-Fluoreszenzreaktion nachwiesen.
   Innerhalb der pflanzenphysiologischen Untersuchungen gelang beispielsweise auch mithilfe der Fluoreszenz-Lebensdauer-Mikroskopie (fluorescent lifetime imaging FLIM) Analyse die Aufnahme von Al³⁺ in Wurzelzellen von *Arabidopsis thaliana* live mitzuverfolgen (Barbourina und Rengel, 2009). Bereits 2000 erschien von Silva und Kollegen eine Arbeit über Sojabohnen (*Glycine max* L. Merr.), die mit Al³⁺ behandelt worden waren und welche die Verteilung der Aluminium-Ionen in der Wurzelspitze durch Lumogallion-Reaktion lokalisierten.
   Kataoka et al., 1997 beschäftigten sich mit der Viabilität und der Wachstumserholung in Tabakpflanzen (*Nicotina tabacum* L.) und Sojabohnen (*Glycine max* (L.) Merr. cv. Tsurunoko) nach Gabe von Aluminiumchlorid AlCl₃. Sie testeten dabei verschiedene Aluminium-färbende Substanzen, welchen kolorimetrische Methoden (Aluminon, Hämatoxylin, Pyrocatechol) oder eine Fluoreszenzfärbung (Lumogallion und Morin) zugrunde lagen und bestätigten den Einsatz von Lumogallion als den empfindlichsten Nachweis bei Verwendung von Epifluoreszenz- und Konfokalmikroskopen zur Analyse des Gehaltes an löslichen Aluminium-Ionen (Kataoka et al., 1997). Ebenfalls in Tabakpflanzen wurde die Wirkung von AlCl₃ auf das Photosystem II mithilfe einer Lumogallion-Reaktion analysiert (Li et al., 2012).
3. Gesteinsproben:
   Fließ-Injektions-Analysen (FIA) mit Fluoreszenzdetektion von Aluminium aus Bodenproben wurden nach Auftrennung der einzelnen wasserlöslichen Aluminium-Arten unter Verwendung von Ionentauschern mittels Lumogallion durchgeführt (Yamada et al., 2002).
   Die Auflösung von gut sowie schlecht kristallisierten Kaoliniten war Gegenstand der Forschung in der Publikation von Sutheimer und Kollegen 1999. Unter Anwendung der Hochleistungskationenaustauschchromatographie (High-Performance Cation Exchange Chromatography), welche eine Detektion von freiem Al³⁺ wie auch von komplexiertem Aluminium bis zu einer Minimumkonzentration von 7 nM ermöglicht (wobei diese Methode nicht geeignet ist, um polymere Aluminiumoxyhydroxide nachzuweisen), wurde nach Gradientenelution unter Einsatz von Lumogallion lösliches Aluminium in den Eluaten identifiziert (Sutheimer et al., 1999).
   Um den Abbau von eingeatmeten Partikeln in der Lunge zu simulieren, wurde Montmorillonit verwendet, welcher in künstlichem Lungenfluid inkubiert und durch diesen partiell aufgelöst wurde. Die Analyse des Gesamtgehaltes an Aluminium des Montmorillonits in Lösung erfolgte anschließend im Fluoreszenzmessverfahren des künstlichen Lungenfluids ebenfalls mit Lumogallion (Ramos Jareño, 2013). Auch in dieser Publikation wurde keine direkte Färbung der Alumosilikate beschrieben.
4. Tier und Mensch:
   Der Nachweis und die Quantifizierung von Aluminium-Ionen in menschlichen Blut- und Urinproben mittels HPLC wurden von Lee et al. 1996 veröffentlicht.
   Die Detektion der Verteilung von Aluminium in Knochen- und Lungengewebe von Wistar Ratten nach intraperitonealer Gabe von Kaliumaluminiumsulfat und von Aluminiumhydroxid in der Nahrung gelang in einem konfokalen Lasermikroskop nach Fixierung und Einbettung der Gewebeproben in Methylmethacrylat-Gießharz und anschließender Färbung von Aluminium-Ionen in der histologischen Probe mit Lumogallion (Uchiumi et al., 1998).
   2005 erschien eine Arbeit von Zhou und Yokel, in der ein Zellkulturmodell der gastrointestinalen Absorption von ²⁷Al als Ion, Zitrat, Maltolat, Fluorid oder Hydroxid untersucht und in der Aluminium während der Analyse am Konfokalmikroskop durch Lumogallion sichtbar wurde.
   Des Weiteren konnte eine Färbung von Aluminium-haltigen Stoffen in lebenden (Mile et al., 2015) und fixierten Zellen sowie histologischen Dünnschnitt-Präparaten durch Fluoreszenzmikroskopie erreicht werden (Mold et al., 2014; 2016 und Mirza et al., 2016; 2017). Hierbei wurden Aluminiumoxidhydroxid-Ionen (AlO(OH)), wie sie in Adjuvantien Verwendung finden, in einer Monozytenzelllinie unter physiologischen Bedingungen (Mile et al., 2015) sowie nach Fixierung der Zellen (Mold et al., 2014 und 2016) identifiziert. Ferner gelang die Detektion von Aluminium-Ionen in mit Histo-Clear entwachsten histologischen Präparaten von Alzheimer Patienten mit Lumogallion (Mirza et al., 2016 und 2017).
   Klein und Kollegen publizierten 2014 Ergebnisse der Untersuchung menschlicher Samenflüssigkeit auf Aluminium. Auch diese Arbeitsgruppe verwendete Lumogallion für Teile ihrer Experimente (Klein et al., 2014).

Trotz des vielfältigen Einsatzes von Lumogallion gab es bislang keine Möglichkeit einer Markierung jedweder Alumosilikatpartikel mit Lumogallion.

Patente, welche die Verwendung von Lumogallion beschreiben, sind beispielsweise:
- Aya Ohkubo, Osamu Shirota, Makoto Sato, Hajime Yoshimura, Erfinder; 2000. Chelate reagent and measuring aluminum and measuring method. Anmeldung: 12.09.2000. US 2004/0101968 A1. 27.05.2004. Unter Verwendung des Chelators 2,2'-dihydroxy-azobenzene, der an Lumogallion einerseits und an die Aluminium-enthaltende Probe andererseits bindet, erfolgt die Bestimmung des Aluminium-Gehaltes ohne Beeinflussung anderer in der Probe anwesenden Substanzen über Chromatographie. Dieses Verfahren inkludiert z.B. folgende Proben: biologische Materialien, Speisen, Getränke, Trinkwasser, Reagenzien, Pharmazeutika, Flusswasser, Seewasser, Meerwasser und Böden und eignet sich im Besonderen zur Messung der Aluminium-Konzentration im Blut von Dialysepatienten im Rahmen labormedizinischer Untersuchungen wie auch zur Bestimmung der Aluminium-Konzentration in pharmazeutischen Produkten. Unabdingbar für die Bestimmung des Aluminium-Gehaltes der Probe ist hierbei, dass eine Reaktionslösung vorhanden ist, welche das Aluminium aus der Probe enthält. Falls für die Aluminium-Analyse nicht eine Flüssigprobe vorliegt, muss diese (z.B. durch Extraktion, Solubilisierung, etc.) vorab hergestellt werden, wobei auch eine Deproteinisierung in biologischen Proben zu erfolgen hat.
- Daido Ishii, Erfinder, 2001. Method for determination of aluminum. Anmeldung: 19.12.2001. US20040259262 A1(. 23.12.2004. Die Messung des Aluminium-Gehaltes über Flüssigkeitschromatographie (HPLC) kann bei einem gepufferten pH-Wert > 6,0 und Raumtemperatur innerhalb von 2 min erfolgen, nicht wie üblich bei saureren pH-Werten mit Inkubation der Probe bei 80 °C für 20 min. Auch bei dieser Erfindung wird der Aluminium-Gehalt der Probe, bei der es sich beispielsweise um ein Pharmazeutikum, pflanzliches oder tierisches Gewebe, Reformkost, Trinkwasser, Tee, ein Kosmetikum, ein alkoholisches Getränk, Leitungswasser, eine Gewässerprobe, Meerwasser, Seewasser, Flusswasser, Industrieabwasser, Brauchwasser, eine Forschungsreagenz, industrielles Rohmaterial, einen Antikörper oder Impfstoff aus einem Antigen, Serum, Urin, Plasma, Blut, Körperflüssigkeit menschlichen oder tierischen Ursprungs, Schweiß, Tränenflüssigkeit, Aszitesflüssigkeit oder Amnionblasenflüssigkeit handeln kann, in einem Lösungsmittel gemessen.

Die Analyse von Fluoreszenzfarbstoffen unter dem Fluoreszenzmikroskop bedingt vielfach die Verwendung von Eindeckmedien ("mounting media"), die ein Ausbleichen ("fading") der Substanzen verhindern.

Gegen das Photobleichen ("photo bleaching") wurde vor allem in Zusammenhang und seit der Verwendung von Immunfluoreszenznachweisen zu Beginn der 1940er Jahre mittels fluoreszierender sekundärer Antikörper (Coons et al., 1941; 1942; 1950) nach chemischen Lösungen für dieses Problem gesucht. Durch die Verwendung von *p*-Phenylendiamin (PPD) in gepuffertem Glyzerin als Eindeckmedium durch G. D. Johnson und Kollegen konnte eine deutliche Verzögerung des Ausbleichens während der Auswertung einer fluoreszierenden Probe unter dem Fluoreszenzmikroskop erzielt werden (Johnson und Nogueira Araujo 1981; Johnson et al., 1982). Davor bestanden Eindeckmedien oftmals nur aus einem 1:9 Gemisch von PBS ("phosphate buffered saline", phosphatgepufferte Kochsalzlösung) oder TRIS ("trisodium phosphate", Trinatriumphosphat) mit Glyzerin (Huff et al., 1982; Valnes und Brandtzaeg, 1985), welches ein rasches Ausbleichen kaum zu verhindern mochte und fotografische Dokumentationen der Auswertungen nahezu unmöglich machte (Huff et al., 1982). Im Gegensatz dazu erwiesen sich mit PPDenthaltendem Eindeckmedium präparierte Proben nicht nur für mindestens eine Woche bei einer Lagerung bei 4 °C stabil bevor ein graduelles Verblassen eintrat, sondern sie konnten auch mit weit höherer Intensität und daher auch einer besseren morphologischen Erkennung unter dem Fluoreszenzmikroskop beobachtet werden (Huff et al., 1982). Weiters zeigte die Verwendung von PPD eine Erhöhung der Fluoreszenz-Aktivität, während sie keinen Einfluss auf eine Antigen-Antikörper-Bindung hatte (Platt und Michael, 1983).

PPD ist beispielsweise in VECTASHIELD Antifade Mounting Medium (VectorLaboratories, USA) enthalten (Longin et al., 1993; Cordes et al., 2011).

Andere Additive in Eindeckmedien können zum Beispiel n-Propylgallat (NPG) und 1,4-Diazobicyclo(2,2,2)-oktan (DABCO) sein (z.B. Valnes und Brandtzaeg, 1985; Longin et al., 1993; Florijn et al., 1995), welche allerdings sehr hohe Konzentrationen benötigen, um effektiv zu sein (Longin et al., 1993), wie auch Ascorbinsäure (AA), 6-Hydroxy-2,5,7,8-tetramethylchroman-2-carbonsäure (Trolox), Mercaptoethylamin (Cysteamin) und Cyclooctatetraen (Cordes et al., 2011).

Der Erfindung liegt die Aufgabe zugrunde, eine schnelle, stabile, nicht toxische und kostengünstige Markierung und Detektion von Alumosilikaten selbst wie auch diese z.B. in histologischen Schnitten ohne zwingend vorangegangene Vorbehandlung wie Entparaffinierung, in Lavagen, geologischen Proben (Mineralpartikeln, Gesteinen, etc.) und Zellkultur-Präparaten zu ermöglichen.

Erfindungsgemäß wird diese Aufgabe durch ein Verfahren mit den Merkmalen des kennzeichnenden Teils des Anspruches 1 gelöst. Mit anderen Worten, dadurch, dass das Eindeckmedium para-Phenylendiamin (1,4-Diaminobenzol) in Glycerin enthält bzw. daraus besteht.

Ein solches überraschenderweise verwendbares Eindeckmedium ist beispielsweise VECTASHIELD Antifade Mounting Medium, VectorLaboratories, USA

Die Erfindung stellt somit ein Verfahren zur spezifischen und stabilen Markierung von Zeolithen, insbesondere von Klinoptilolith, in Zellkulturen und Gewebeschnitten, aber auch der Partikel selbst dar, die sich durch ihre rasche, hochreproduktive, kostengünstige und technisch unkomplizierte Handhabung auszeichnet.

Die beigefügte Zeichnung stellt dabei in:

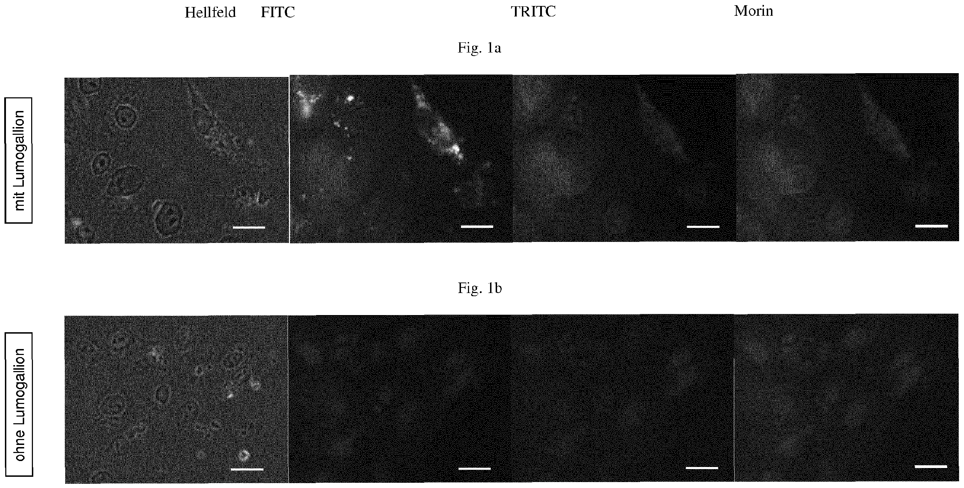

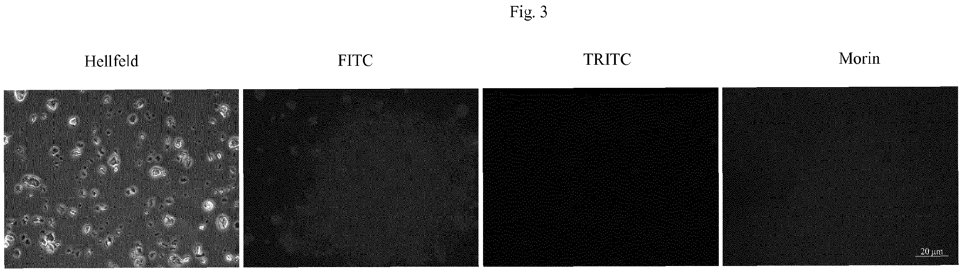

Figs. 1, 2, 4, 5 und 6 Beispiele für die Färbung und in Fig. 3 ein Beispiel ohne auswertbare Färbung dar, werden weiter unten genauer erläutert.

Detaillierter gesagt, ist für die Anwendung der Markierung der Alumosilikate alleine oder in biologischen Präparaten mittels Lumogallion von Vorteil, sowohl die Dauer und die Inkubationstemperatur, als auch die Konzentrationen, die pH-Werte der verwendeten Puffer und Lösungen sowie die Partikelanzahl gemäß nachfolgender Aufstellung einzuhalten:
a. Zeolithe oder andere Alumosilikate: 10 µg/cm²
b. Partikelgröße im Durchmesser: zirka 1 - 60 µm
c. Lumogallion-Lösung: 10 - 200 µM in 50 mM HEPES-Puffer pH 7,2 oder 100 - 200 µM in 20 - 50 mM Azetat-Puffer, pH 4,0.
d. Inkubationszeit: 2 h - 24 h
e. Inkubationstemperatur: 20 °C - 37 °C / 50 °C - 80 °C

Versuchstechnisch kann die Fluoreszenz-Reaktion bei einer (Raum-)Temperatur bis 37 °C über 12 bis 24 Stunden ("über Nacht") sowie auch bei 50 °C bis 80 °C innerhalb von 2 bis 4 Stunden erfolgen.

Da die Markierung mittels Lumogallion als nicht toxisch bekannt ist, ist auch für den Experimentator ein komfortabler und unproblematischer Umgang während der Herstellung der Lösungen und des Prozesses selbst bei Einhaltung aller Schutzvorkehrungen, die für ein Arbeiten im Labor und mit den benötigten Substanzen geboten sind, möglich.

### DETAILLIERTE BESCHREIBUNG:

Zellkultur-Monolayer:
   In Paraformaldehyd-fixierten Zellkulturen, welche zuvor mit Alumosilikaten oder Alumosilikaten in Gemischen mit Nicht-Alumosilikaten behandelt wurden, lassen sich die Alumosilikate eindeutig nachweisen.
   Eine etwaige Autofluoreszenz der Zellen wird durch die Behandlung mit Lumogallion überraschenderweise verringert und die markierten Partikel werden unverwechselbar erkennbar.
   Ein besonderer Vorteil dieser spezifischen Markierung von Alumosilikat-Partikeln mittels Lumogallion stellt die überraschend hohe Stabilität der Fluoreszenz dar, welche durch die Verwendung eines antioxidativen Eindeckmediums noch zusätzlich verlängert werden kann, sodass selbst nach mehreren Tagen bis Monaten die Proben nach Aufbewahrung bei 4 °C im Mikroskop ohne starken Qualitätsverlust auswertbar bleiben. Mehrere Beispiele dieser Anwendung stellen die Figuren 1 und 5 sowie 6b dar.
Gewebeproben:
   Histologische Schnitte von Paraffin-eingebetteten, Zeolith enthaltenden Proben (mit einer Dicke von 5 µm - 25 µm), lassen sich überraschenderweise ohne zuvor erfolgte Entparaffinierung ebenfalls mit einer gepufferten Lumogallion-Lösung färben.
   Wie bei Zellkultur-Monolayern wird auch hier die Autofluoreszenz der Gewebsschichte unterdrückt ("gequencht") und die Zeolithe werden spezifisch sichtbar.
   Eine Besonderheit dieser Methode liegt in der überraschend hohen Stabilität der Markierung und physikochemischen Integrität des Fluorophors (Lumogallion) nach der Fixierung und Einbettung, welche zusätzlich unter Verwendung eines antioxidativen Eindeckmediums erfolgen kann. Dieses wirkt einem etwaigen raschen Ausbleichen des Fluorophors durch das Anregungslicht während einer Analyse im Fluoreszenzmikroskop ("Photobleichung") entgegen. Es zeigte sich, dass die Proben längere Zeit ohne signifikanten Verlust der Fluoreszenz unter dem Mikroskop beobachtet bzw. bei 4 °C gekühlt gelagert (mehrere Tage bis Monate) werden können.

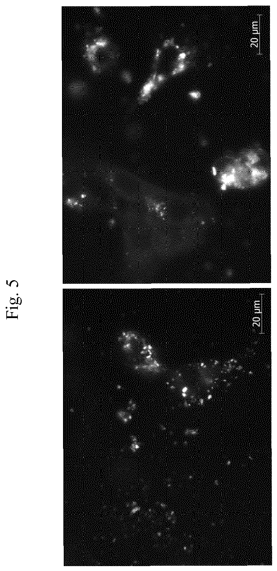

   Fig. 5 zeigt eine Auswertung von Proben, die nach erfolgter Lumogallion-Färbung 8 Wochen im Kühlschrank bei 4 °C gelagert worden waren und deren Fluoreszenzmarkierung nach dieser Zeitspanne noch immer ohne signifikanten Qualitätsverlust deutlich abgegrenzt und intensiv leuchtend zu erkennen ist.
Mineralogisch-geologische Proben:
   Die Markierung von Aluminium in Alumosilikaten ermöglicht Zeolithe nicht nur in biologischen Proben nachzuweisen, sondern die selbigen auch in Gesteinsproben - allerdings überraschenderweise nur nach Einbettung in Paraffin/Paraffinersatz - von anderen Mineralien durch Lumogallion-Färbung zu unterscheiden. Diese Einbettung stellt sich dabei als entscheidender und nicht zu umgehender, sowie bis dato noch nie beschriebener Schritt im Nachweisprozess dar (siehe Fig. 2), ohne den keine auswertbare Markierung mit Lumogallion erfolgt (siehe Fig. 3). Eine Zusammenfassung von Beispielen verschiedener Gesteine, die eine bzw. keine Färbung mit Lumogallion zeigen, findet sich in Fig. 6a.
   Die Färbung von Aluminium mit Lumogallion bietet den Vorteil, unter physiologischem pH (7,0 - 7,2) und unter Verwendung üblicher (Zellkultur-)Puffer (wie HEPES oder PIPES) und Medien durchführbar zu sein. Alternativ können die Präparate auch im sauren Bereich und mit anderen üblichen Puffern wie beispielsweise 20 mM Azetat-Puffer pH 4,0 inkubiert werden. Das breite Spektrum an Puffern und pH-Werten ist in der praktischen Anwendung äußerst hilfreich.
   Zur Detektion der Fluoreszenz, die aus der Reaktion von Lumogallion mit Aluminium aus der Probe entsteht, erfordert es keine nur für diesen Zweck nötigen Spezialapparaturen, sondern es können handelsübliche Epifluoreszenz- und Konfokalmikroskope sowie Spektrophotometer mit den entsprechenden Filtern eingesetzt werden.
   Aufgrund der relativ hohen Stabilität der Markierung nach der Fixierung und Einbettung unter Verwendung eines Eindeckmediums können die Proben ohne signifikanten Qualitätsverlust länger unter dem Mikroskop beobachtet bzw. gelagert (mehrere Tage bis Monate) werden, dies zeichnet die Erfindung besonders aus (als Beispiel Fig. 5).

Die Erfindung wird im Folgenden näher beschrieben, wobei die einzelnen Figuren folgendes zeigen:

### Fig. 1 - Lumogallion-Färbung von Zeolith in fixierten Zellkulturen

Die Auswertung der Proben wurde an einem Epifluoreszenzmikroskop unter Verwendung spezieller Filter (siehe Tabelle 2) vorgenommen.

Die Anregung erfolgte bei zirka 500 nm, die Emission wurde bei rund 570 nm detektiert.

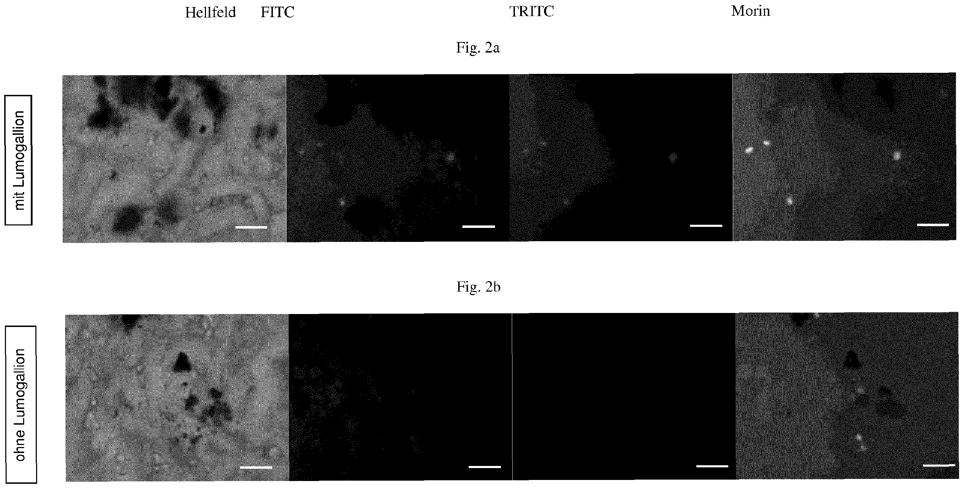

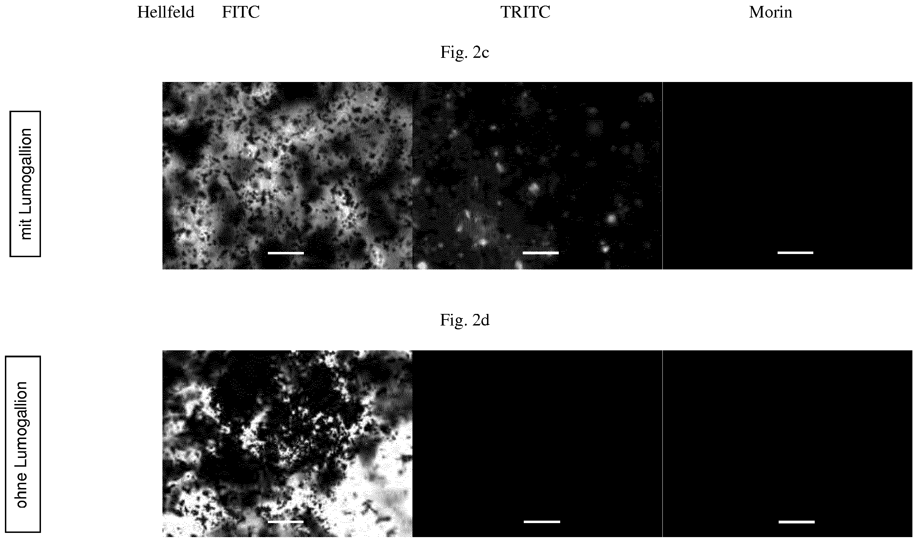

### Fig. 2 - Gemeinsame Einbettung verschiedener Partikel (Zeolith und Aktivkohle oder Zeolith und Silizium) mit anschließender Lumogallion-Färbung

Gemeinsame Einbettung unterschiedlicher Gesteinsproben, welche entweder eine oder keine Fluoreszenzfärbung mit Lumogallion zeigten.

Die Spezifität der Lumogallion-Markierung für Aluminium-haltige Verbindungen wird ersichtlich, ebenso die Spezifität der einzelnen Filter für unterschiedliche Gesteine.

Fig. 3 - **Partikelfärbung mit Lumogallion ohne vorangegangene Einbettung** Durchgeführte Färbung von Zeolith-Partikeln ohne vorangegangene Einbettung in Paraffin/Paraffinersatz.

Es konnte keine Markierung in der Probe detektiert werden.

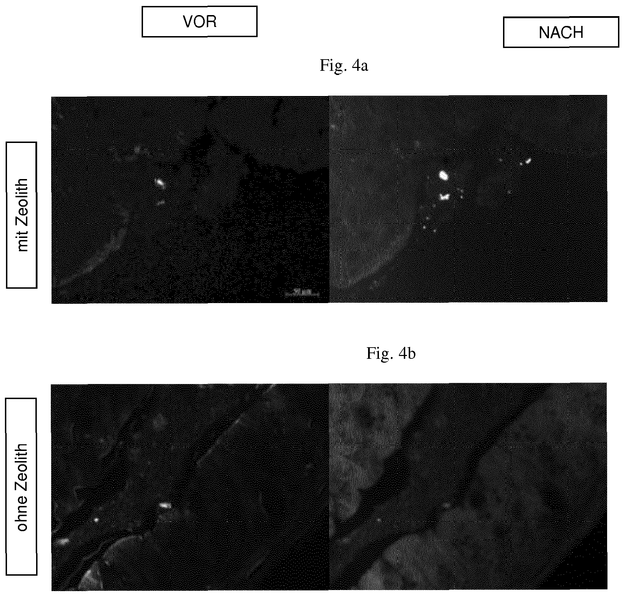

### Fig. 4 - Mit Lumogallion gefärbte histologische Proben

In Paraffin eingebettete, nicht entwachste Darmgewebe-Proben von mit und ohne Zeolith-Beimischung im Futter ernährten Mäusen nach Färbung mit Lumogallion.

### Fig. 5 - Zeolith-behandelte, fixierte und mit Lumogallion markierte Zellen, die 8 Wochen bei 4 °C gelagert worden waren

Die Proben waren nach der ersten Auswertung unverändert für 56 Tage im Kühlschrank bei 4 °C aufbewahrt worden, bevor sie neuerlich begutachtet wurden.

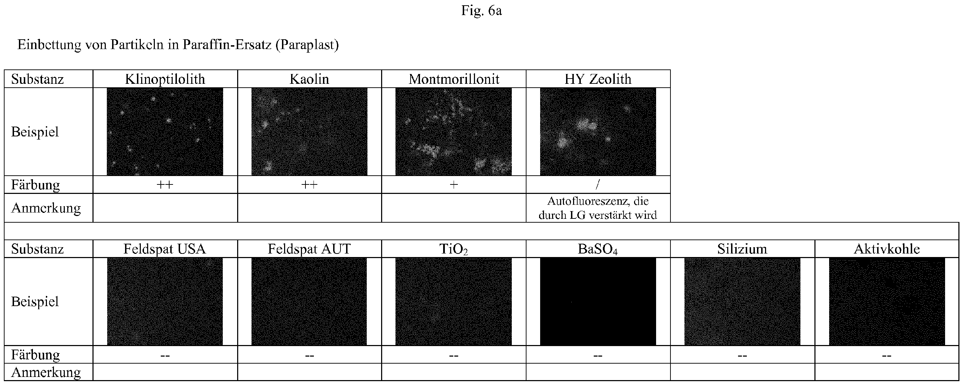

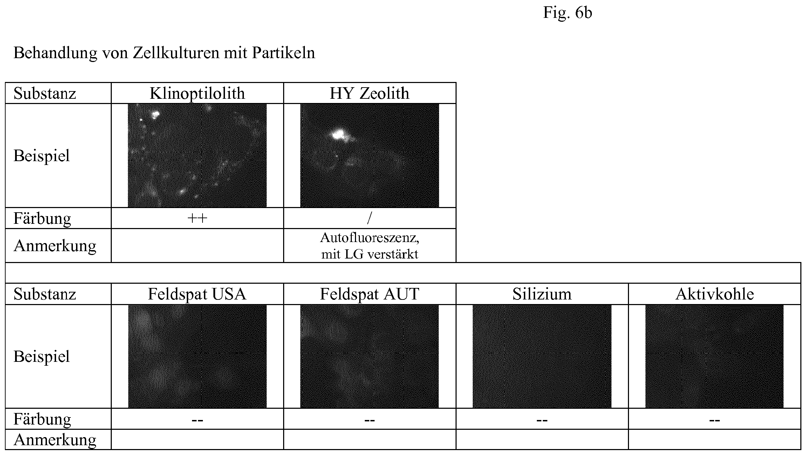

Fig. 6 - **Fluoreszenz-Aufnahmen der in Tabelle 1 zusammengefassten Testsubstanzen** Überblick über unterschiedliche in Paraffin-Ersatz (Paraplast) eingebettete oder für Zellkultur-Behandlungen verwendete Gesteinsproben, die positiv mit Lumogallion färbten oder nicht mit Lumogallion markiert werden konnten.

In den Figuren 1 - 5 wurde Klinoptilolith (aufgereinigt ("purified") nach US 8173101 B2) verwendet, der ein Alumosilikat vulkanischen Ursprungs ist und innerhalb der Tektosilikate zur Gruppe der Zeolithe zählt.

Figur 6 ermöglicht einen Vergleich von Klinoptilolith mit dem künstlich hergestellten HY Zeolith einerseits bei der Verwendung in Zellkulturen und andererseits nach Einbettung in Paraffin-Ersatz (Paraplast).

Im Detail stellen die Figura dar:
**Fig. 1** zeigt eine typische Auswertung am Epifluoreszenzmikroskop mit 100facher Vergrößerung (Axiovert 200M, Firma ZEISS) einer Anwendung der Lumogallion-Färbung von Paraformaldehyd-fixierten menschlichen Zellkulturen (MCF-10A, ATCC CRL-10317) mit (Fig. 1a) und ohne (Fig. 1b) Klinoptilolith-Behandlung. Klinoptilolith ist ein Alumosilikat vulkanischen Ursprungs und zählt innerhalb der Tektosilikate zur Gruppe der Zeolithe. Die Proben waren zuvor über Nacht bei 37 °C mit 100 µM Lumogallion in 50 mM HEPES-Puffer pH 7,2 inkubiert worden. Zwecks Minimierung des Verblassens der Fluoreszenz-Färbung wurde ein antioxidatives Eindeckmedium (VECTASHIELD Antifade Mounting Medium, Vector Laboratories, USA) verwendet. Zur Analyse eingesetzt wurden die in Tabelle 2 beschriebenen Filter. Die Klinoptilolith-Partikel fluoreszieren deutlich und sind zum Teil sogar einzeln im FITC-Filter erkennbar (Fig. 1a). Die unbehandelten Klinoptilolith-Partikel der Kontrolle zeigen keine Autofluoreszenz (Fig. 1b). Der Maßstabsbalken entspricht 20 µm.
**Fig. 2** bildet eine gemeinsame Einbettung von Klinoptilolith mit nicht mit Lumogallionfärbbaren Partikeln ab. Die Partikel (Klinoptilolith und Aktivkohle, Fig. 2a und 2b, oder Klinoptilolith und Silizium, Fig. 2c und 2d) wurden hierfür in Paraffin-Ersatz (Paraplast X-tra Tissue Infiltration/Embedding Medium, McCormick Scientific, PA) eingebettet und nachfolgend am Mikrotom bei Raumtemperatur mit einer Präparatdicke von 16 µm geschnitten. Zur Färbung mit 100 µM Lumogallion in 50 mM HEPES-Puffer pH 7,2 wurde der Schnitt über Nacht bei 37 °C inkubiert (Fig. 2a und 2c). Fig. 2b und 2d zeigen die dazugehörigen Kontrollen, die gleich wie die Proben behandelt wurden - allerdings erfolgte die Inkubation über Nacht nur in 50 mM HEPES-Puffer ohne Lumogallion. Kontrollen wie auch gefärbte Proben wurden mit Eindeckmedium (VECTASHIELD Antifade Mounting Medium, Vector Laboratories, USA) vor dem Ausbleichen geschützt. Die in den Figuren 2a und 2b wie auch 2c und 2d verwendeten Proben sind jeweils direkt aufeinanderfolgende Serienschnitte, wobei unterschiedliche Bereiche in den Figuren abgebildet werden. Wie in Fig. 2a und 2c klar erkennbar, ist eine Auswertung am Epifluoreszenzmikroskop mit 100facher Vergrößerung (Axiovert 200M, Firma ZEISS) und im Falle des Aktivkohle-Klinoptilolith-Gemisches mit allen in Tabelle 2 beschriebenen Filtern problemlos möglich - die Färbung des Klinoptiloliths ist kräftig, während die Aktivkohle nicht fluoresziert. Ähnlich verhielt es sich mit einem Bariumsulfat-Klinoptilolith-Gemisch (nicht abgebildet). Das Silizium-Klinoptilolith-Gemisch jedoch konnte nur mit den FITC- und TRITC-Filtern analysiert werden, da die für eine Auswertung mittels Morin-Filters nötige Anregung im UV-Licht-Bereich die Silizium-Partikel erwärmte, wodurch das Paraplast schmolz und die Partikel zu diffundieren begannen, was eine Detektion unmöglich machte. Dies veranschaulicht eindrucksvoll die hohe Spezifität der Markierung von Aluminium durch Lumogallion und die Notwendigkeit der Verwendung von geeigneten Filtern, welche spezifisch für die einzelnen Proben zu wählen sind. Der Maßstabsbalken entspricht 20 µm.
**Fig. 3** stellt eine Partikelfärbung von Klinoptilolith ohne vorangegangene Einbettung in Paraffin oder Paraplast im Hellfeld und unter Verwendung verschiedener Filter (FITC, TRITC, Morin) dar. Die Proben wurden mit einer Partikel-Konzentration von 133 µg/ml mit 100 µM Lumogallion in 50 mM HEPES-Puffer pH 7,2 bei 37 °C über Nacht inkubiert, anschließend zentrifugiert und das Pellet in 15 µl Eindeckmedium (VECTASHIELD Antifade Mounting Medium, Vector Laboratories, USA) auf einem Objektträger fixiert. Die Markierung der Klinoptilolith-Partikel mit Lumogallion ist am Epifluoreszenzmikroskop mit 100facher Vergrößerung (Axiovert 200M, Firma ZEISS) kaum bis gar nicht detektierbar und daher auch nicht auswertbar. Der Maßstabsbalken entspricht 20 µm.
**Fig. 4** veranschaulicht murine, nicht entparaffinierte, histologische Darm-Proben einer mit Klinoptilolith gefütterten Maus (Fig. 4a) und einer Kontroll-Maus ohne Klinoptilolith-Beimengung im Futter (Fig. 4b). Die Gewebeschnitte wurden zuvor unbehandelt (Fig. 4a und 4b "VOR") und anschließend mit Lumogallion gefärbt (Fig. 4a und 4b "NACH") am Epifluoreszenzmikroskop mit 32facher Vergrößerung (Axiovert 200M, Firma ZEISS) aufgenommen. Der Maßstabsbalken entspricht 50 µm. Die Proben waren nach der Entnahme, Fixierung und nachfolgenden Paraffin-Einbettung zirka 2,5 Jahre bei Raumtemperatur gelagert worden bevor sie geschnitten (zirka 15 µm) und für die Lumogallion-Färbung eingesetzt wurden. Die Schnitte wurden bei 37 °C mit 100 µm Lumogallion in 50 mM HEPES-Puffer pH 7,2 über Nacht inkubiert und dann mit Eindeckmedium (VECTASHIELD Antifade Mounting Medium, Vector Laboratories, USA) auf einem Objektträger fixiert. Die Färbung der Partikel ist eindeutig erkennbar (Fig. 4a "NACH"). Die lange Lagerdauer der histologischen Schnitte minimierte die Qualität der Lumogallion-Markierung der Klinoptilolith-Partikel nicht. Die Fig. 4b "NACH" veranschaulicht das Quenchen der Autofluoreszenz durch die Inkubation mit Lumogallion.
**Fig. 5** gibt zwei Beispiele von Fluoreszenz-Aufnahmen einer Auswertung von mit Lumogallion markierten Proben nach einer längeren Lagerzeit (8 Wochen bei 4 °C) wieder. Die menschlichen Zellkulturen (MCF-10A, ATCC CRL-10317) waren nach Klinoptilolith-Behandlung mit Paraformaldehyd fixiert und nachfolgend mit Lumogallion inkubiert worden. Die mit Klarlack versiegelten Proben verblieben nach der ersten Analyse ohne weitere Manipulation 2 Monate unter kontinuierlicher Kühlung ungeöffnet, bevor eine neuerliche Analyse (Fig. 5) durchgeführt wurde. Die Auswertung erfolgte mit 100facher Vergrößerung (Axiovert 200M, Firma ZEISS) unter Verwendung des FITC-Filters. Die Klinoptilolith-Partikel fluoreszieren unverändert intensiv - die Fluoreszenz erscheint durch die lange Lagerung nicht feststellbar verringert. 100x Vergrößerung. Der Maßstabsbalken entspricht 20 µm.
**Fig. 6** zeigt eine Zusammenfassung der verwendeten Testsubstanzen und deren Reaktion auf eine Lumogallion-Inkubation. Während in Fig. 6a die in Paraplast (Paraffin-Ersatz) eingebetteten Partikel dargestellt werden, fasst Fig. 6b Zellkulturen, die mit Alumosilikat- oder Nicht-Alumosilikat-Partikeln inkubiert, anschließend fixiert und letztlich mit Lumogallion behandelt worden waren, zusammen. Die Auswertung der Proben erfolgte unter immer gleichen Bedingungen (FITC-/TRITC-/Morin-Filter, Axiovert 200M, Firma ZEISS) und mit selber Vergrößerung (100fach). Die Intensität der Färbung wurde wie folgt angegeben: "++" bedeutet starke, "+" schwache und "--" keine Markierung der Partikel mit Lumogallion. Das Symbol "/" wurde verwendet, um experimentelle Probleme anzuzeigen, welche in der Rubrik "Anmerkung" näher ausgeführt werden; in den angeführten Beispielen handelte es sich konkret um eine starke Autofluoreszenz der Testsubstanz.

Ein spezifischer Nachweis von Alumosilikatverbindungen in Zellen (Fig. 1, 5 und 6b) und Geweben (Fig. 4) ermöglicht die Identifikation und Verteilung derselbigen und somit erstmals eine Auffindung und Nachverfolgung von diesen Partikeln nach deren Anwendung. Von großem Vorteil ist dabei auch die Möglichkeit einer nachträglichen Markierung von Alumosilikaten in biologischem Material, da die Lumogallion-Reaktion auch in Paraformaldehyd-fixierten Präparaten ohne Qualitätsverlust der Markierung durchführbar ist. Eine Entwachsung von in Paraffin/Paraffin-Ersatz eingebetteten Proben ist für das Gelingen der Färbung überraschenderweise nicht nötig.

Weiters wird durch die Färbung mit Lumogallion eine etwaig vorhandene Autofluoreszenz der Zellen beziehungsweise des Zellverbandes überraschenderweise gequencht (Fig. 4b "NACH"), was zusätzlich zu einer deutlichen Identifikation der Zeolith-Partikel beiträgt.

Die Markierung der Partikel erweist sich überraschenderweise als sehr stabil, sodass diese auch nach Tagen bis Monaten eindeutig erkennbar sind (Fig. 5).

Die Möglichkeit der Markierung von Zeolith-Partikeln in einem breiten Temperaturbereich und bei variablen pH Werten erweitert das Anwendungsspektrum deutlich und trägt zu einer vielfältigen Applikation bei.

Eine Färbung von Aluminosilikat-Partikeln alleine mit Lumogallion ist ebenso möglich (Fig. 6a) - überraschenderweise jedoch nur, wenn diese vorab in Paraffin oder Paraffinersatz eingebettet wurden (vergleiche Fig. 3, die keine Färbung von Partikeln mit Lumogallion ohne Paraffin bzw. Paraplast zeigt). Dadurch können Zeolithe auch von anderen Substanzen bzw. Mineralien unterschieden werden - Beispiele dafür sind eine gemeinsame Einbettung von Klinoptilolith und Aktivkohle (Fig. 2a und 2b) oder Klinoptilolith und Silizium (Fig. 2c und 2d) oder ebenso Klinoptilolith und Bariumsulfat (nicht gezeigt), wobei jeweils nur der Klinoptilolith gefärbt wurde, während die Aktivkohle (Fig. 2a), das Silizium (Fig. 2c) oder das Bariumsulfat nicht-fluoreszierend verblieben.

Eine neuerliche Färbung von ausgebleichten Partikeln ist im Bedarfsfall ohne Einschränkung möglich und dienlich, falls bereits zuvor markierte Proben nochmals analysiert werden müssen. Hierzu werden die Proben kurz dreimalig in jenem Puffer gespült, in dem die nachfolgend verwendet werdende Lumogallion-Lösung zubereitet wurde (z.B. HEPES- oder Azetat-Puffer) und anschließend in der Lumogallion-Markierungslösung inkubiert. Die Inkubationsdauer und -temperatur kann dabei jener der primären Inkubation entsprechen, ebenso die weitere Vorgangsweise bis zur Einbettung (siehe Ausführungsbeispiele).

Zusätzlich hat die Markierung mit Lumogallion den Vorteil, über ein breites Spektrum hinweg detektierbar zu sein, sodass die gebräuchlichsten Filter (siehe Tabelle 2) Anwendung finden können. Hierbei empfiehlt es sich, den für die jeweilige Testreihe am besten geeigneten Filter auszutesten - Mineralien bzw. Gesteine weisen unterschiedliche Fluoreszenz-Maxima/Fluoreszenzspektra bei Lumogallionfärbung auf und die Wahl der Filter sollte möglichst dahingehend berücksichtigt werden (zum Beispiel Fig. 2c - hier lassen sich die Siliziumpartikel im Morin-Filter nicht nachweisen).

Der Anwender kann mit den in einem zellbiologischen Labor üblichen Geräten arbeiten; eine Anschaffung spezieller, nur für die Lumogallionfärbung hergestellter Arbeitsmittel entfällt.

Zusammengefasst zeichnet sich somit die Erfindung durch ihre rasche und unkomplizierte Durchführbarkeit, ihre Spezifität für Alumosilikate, ihre hohe Reproduzierbarkeit und Stabilität über mehrere Tage bis Monate, sowie durch das Entfallen der Entparaffinierung von histologischen Präparaten und der Möglichkeit einer neuerlichen/wiederholten Färbung mit Lumogallion aus.

Von besonderem Nutzen könnte sich diese Eigenschaft innerhalb der forensischen Analyse von Bodenproben erweisen (Tibet und Carter, 2008). Auch ist eine Identifikation von Alumosilikaten in menschlichen und tierischen Histopräparaten möglich, welche in weit zurückliegender Zeit angelegt wurden und somit neue Erkenntnisse - ohne hohe zusätzliche Kosten und großem Zeitaufwand - bringen.

Des Weiteren lassen sich ebenso spezifisch Alumosilikate in Mineralgemischen/Gesteinsgemischen identifizieren und in der Folge auch die Menge an Alumosilikaten in der zu prüfenden Substanz definieren.

Innerhalb kurzer Zeit und mit geringen technischen Hilfsmitteln wie auch personellen Anforderungen gelingt durch diese Methode ein hochreproduzierbarer Nachweis von Alumosilikaten im Rahmen eines analytisch-diagnostischen Schnellnachweises oder zur bildlichen Darstellung mithilfe eines Fluoreszenzmikroskops und der dafür geeigneten Filter.

### AUSFÜHRUNGSBEISPIELE:

### Beispiel Markierung von Aluminium in Zellkultur-Proben:

Probenvorbereitung:
   - Die unterschiedlichen Gesteinsproben (siehe Tabelle 1) wurden in Wachstumsmedium mit Antibiotika (Penicillin/Streptomycin) auf eine Endkonzentration von C_{E} =̂ ≈ 100 mg/ml verdünnt.
      Diese Suspension diente als Stammlösung und wurde bei 4 °C gelagert.
Versuchsablauf:
   - Einen definierten Zeitraum vor Versuchsbeginn wurden Zellen einer ausgewählten Kultur (zum Beispiel einen Tag vor dem Start des Experiments falls MCF-10A Zellen, ATCC CRL-10317, Verwendung fanden, oder 5 Tage im Falle von Caco-2 Zellen, ATCC HTB-34) auf in Petri-Schalen befindliche sterile runde Glasplättchen (Durchmesser 10 mm) ausgesät.
   - Nach 24 Stunden bei Wachstumskonditionen wurden die Zellen (jeweils 1 Glasplättchen pro Ansatz) mit 10 µg/cm² einer der Testsubstanzen (siehe Tabelle 1) bei Wachstumsbedingungen für weitere 24 Stunden inkubiert.
      Die Glasplättchen wurden anschließend einmal mit 50 mM HEPES-Puffer pH 7,2 gewaschen und für 30 min bei Raumtemperatur in 0,5 % - 2,0 % Paraformaldehyd in 50 mM HEPES-Puffer pH 7,2 fixiert.
   - Danach wurden die Glasplättchen neuerlich 3x in oben genanntem HEPES-Puffer gewaschen, bevor sie in 1 ml einer
      a) 100 µM Lumogallion-Lösung in 50 mM HEPES-Puffer pH 7,2 oder
      b) 200 µM Lumogallion-Lösung in 20 mM Azetat-Puffer pH 4,0 für entweder
         ∘ 2 bis 4 Stunden bei 60 °C bis 80 °C
            oder
         ∘ 12 bis 24 Stunden bei Raumtemperatur bis 37 °C
         stets lichtgeschützt inkubiert wurden.
   - Vor der Einbettung in Eindeckmedium (VECTASHIELD Antifade Mounting Medium, Vector Laboratories, USA) wurden die Glasplättchen kurz in doppelt destilliertes Wasser getaucht und an einem sauberen Papiertuch abgetropft.
   - Die eingebetteten Proben wurden mithilfe eines zähflüssigen Klarlacks luftdicht mit dem Objektträger verbunden und so vor Austrocknung geschützt.
   - Die einzelnen Proben konnten danach mithilfe eines Epifluoreszenzmikroskops und unterschiedlicher Filter (TRITC-, FITC- oder Morin-Filter, siehe Tabelle 2) auf eine Markierung mit Lumogallion hin untersucht werden (siehe Fig. 1 und 6b).

### Beispiel Markierung von Alumosilikaten in Histoschnitten:

Versuchsablauf entparaffinierte Proben:
1. Die in Paraffin oder Paraffin-Ersatz (Paraplast) eingebetteten Gewebeproben wurden mit einer Präparatdicke von 5 µm bis 25 µM am Kryomikrotom geschnitten und auf Glasobjektträger transferiert.
2. Anschließend wurden die Schnitte 3x mit PBS gewaschen.
3. Es folgte eine Inkubation in Neo-Clear (Xylol-Ersatz für die Mikroskopie, Merck, Deutschland) für 3 min, leicht schüttelnd.
4. Dann wurde das verwendete Neo-Clear entfernt und durch frisches Neo-Clear ersetzt, welches wiederum leicht schüttelnd auf der Probe verblieb. Diese Inkubation dauerte 1 min lang.
5. Hernach wurde der Schnitt mehreren Waschschritten unterzogen:
   ∘ 3x mit PBS
   ∘ 3x mit EtOH_{absolut}
   ∘ 3x mit 50 mM Hepes pH 7,2
6. Daran schloss die Inkubation mit
   a) 100 µM Lumogallion in 50 mM Hepes-Puffer pH 7,2
      oder
   b) 200 µM Lumogallion in 20 mM Azetatpuffer pH 4,0
   für 12 bis 24 Stunden ("über Nacht") bei 37 °C an.
7. Um eine längere Haltbarkeit der Fluoreszenzfärbung zu erzielen, wurden die Schnitte mit Eindeckmedium (zum Beispiel VECTASHIELD Antifade Mounting Medium, Firma Vector Laboratories, USA) überschichtet und mit einem Deckgläschen abgedeckt.
8. Die eingebetteten Schnitte wurden vor Austrocknung geschützt, indem unter Verwendung eines zähflüssigen Klarlacks das Deckgläschen mit dem Objektträger verbunden wurde.
9. Die einzelnen Proben konnten danach mithilfe eines Fluoreszenzmikroskops und unterschiedlicher Filter (TRITC-, FITC- oder Morin-Filter, siehe Tabelle 2) auf eine Markierung mit Lumogallion hin untersucht werden.

Versuchsablauf paraffinierte Proben:
Die Paraffin-Schnitte wurden wie im oben angeführten Protokoll hergestellt (Punkt 1), auf den Objektträgern gewaschen (Punkt 5), mit Lumogallion inkubiert (Punkt 6), eingebettet (Punkt 7 und 8) und unter dem Epifluoreszenzmikroskop analysiert (Punkt 9). Die Punkte 2, 3 und 4 entfielen dabei. Als ein Beispiel der Anwendung dient Fig. 4.

### Beispiel Markierung von Alumosilikaten in mineralogisch-geologischen Proben:

1. Die Glasobjektträger wurden mit 70 % Ethanol gereinigt um ein Haften des Liquid Blocker Super PAP-Pens (Liquid-Repellent Slide Marker Pen, Science Services, Deutschland) zu ermöglichen. Mit diesem wurden Rechtecke auf das Glas gezeichnet, die nach deren Trocknung als "Wannen" zur Befüllung mit Puffer oder mit Lumogallion-Lösung dienen sollten.
2. Die zu feinen Partikeln (zirka 1 µm bis 60 µm im Durchmesser) vermahlenen Gesteinsproben (siehe Tabelle 1) wurden in Paraffin-Ersatz (Paraplast X-tra Tissue Infiltration/Embedding Medium, McCormick Scientific, PA) eingebettetet und mit einer Präparatdicke von 14 µm bis 16 µm (ähnlich wie die der Gewebeproben) am Kryomikrotom geschnitten, bevor sie auf die zuvor präparierten Glasobjektträger transferiert wurden.
3. Die in der Puffer- oder Lumogallion-Lösung schwimmenden Schnitte wurden bei 37 °C für 12 - 24 Stunden inkubiert.
4. Anschließend wurden sie auf neue Objektträger aufgebracht, mit VECTASHIELD Antifade Mounting Medium (Vector Laboratories, USA) überschichtet und mit einem Deckgläschen bedeckt.
5. Eine Versiegelung der Ränder zwischen Objektträger und Deckgläschen mit durchsichtigem Klarlack wurde vorgenommen.
6. Nach der Trocknung des Lackes konnten die Proben im Epifluoreszenzmikroskop betrachtet werden (siehe Fig. 6a - Einzelsubstanzen und Fig. 2 - Gemische). Verwendung dabei fanden die in Tabelle 2 beschriebenen unterschiedlichen Filter (TRITC-, FITC- oder Morin-Filter).

### Tabelle 1

**Tabelle 1: Verwendete Testsubstanzen (Auswahl) und deren Hersteller bzw. Vertreiber.**

| **Substanz** | **Produktinformation/(Produktnummer)** |
|---|---|
| | |
| Aktivkohle | Aktivkohle rein Merck (102183) |
| Aluminiumoxid | Aluminiumoxid Sigma-Aldrich (342750) |
| Bariumsulfat | *Hergestellt aus* Barium chloride dihydrate 99.995 % Suprapur Sigma-Aldrich (101716) *und* Natriumsulfat wasserfrei zur Analyse ACS,ISO,Reag. Ph Eur Merck (106649) |
| Calciumcarbonat | Calciumcarbonat gefällt reinst Ph.Eur., USP AppliChem (A0774) |
| Feldspat AUT | Feldspat trocken Amberger Kaolinwerke (FS900 SF Hirschau) |
| Feldspat USA | Feldspat Fortispar K-30 I Minerals Inc. (ULTRA HalloPure) |
| Halloysit | Halloysite I Minerals Inc. (ULTRA HalloPure) |
| Klinoptilolith | Glock Health, Science and Research (018-01-08-2-1-1) *(Beispiel für einen natürlichen Zeolith, dessen SchwermetallIonen nach* US 8173101 B2 *gegen Kalzium ausgetauscht (*"*exchanged*"*) wurden.)* |
| Kaolin | Kaolinschamotte Amberger Kaolinwerke (AS45 6.400 Hirschau) |
| Montmorillonit | Montmorillonite naturally occurring mineral Alfa Aesar / VWR (42531.22) |
| Silizium | Silicon powder, APS 1-5 micron, 99.9% (metals basis) Alfa Aesar /Thermo Fischer (Kandel) (44185) |
| Titandioxid | Titanium(IV)oxide, anatase Sigma-Aldrich (248576) |
| Zeolith HY | Zeolyst International (CBV400) *(Beispiel für einen künstlich synthetisierten Zeolith.)* |

### Tabelle 2:

**Tabelle 2: Verwendete Fluoreszenz-Filter.**

| Filter | λ_{Absorption} [nm] | | λ_{Emission} [nm] | |
|---|---|---|---|---|
| TRITC | 546/12 | (band pass) | 575 - 640 | (band pass) |
| | 560 | (beam splitter) | | |
| FITC | 450 - 490 | (band pass) | 515 - 565 | (band pass) |
| | 510 | (beam splitter) | | |
| Morin | 433/24 | (band pass) | > 473 | (long pass) |
| | 465 | (beam splitter) | | |

Die erfindungsgemäß erstellten Proben können mithilfe eines Eindeckmediums und unter Verwendung eines Fluoreszenzmikroskopes und unterschiedlicher Filter (TRITC, FITC, Morin) für einen Zeitraum von Tagen bis zu mehreren Monaten stabil detektiert und von einer Vielzahl an Nicht-Alumosilikaten unterschieden werden. In zellbiologischen und histologischen Proben, welche entweder in Paraffin oder Paraffin-Ersatz (Paraplast o.ä.) eingebettet worden waren, können die Proben aufgrund des erfindungsgemäßen Verfahrens insbesondere auch ohne Entparaffinierung markiert werden.

Damit ist gegenüber dem Stand der Technik ein rasches, einfaches, zuverlässiges und kostengünstiges Verfahren gegeben.

In der Beschreibung und den Ansprüchen bedeutet "im Wesentlichen" eine Abweichung von bis zu 10 % des angegebenen Wertes, wenn es physikalisch möglich ist, sowohl nach unten als auch nach oben, ansonsten nur in die sinnvolle Richtung, bei Gradangaben bezüglich der Temperatur sind damit ± 10 °C gemeint.

Alle Mengenangaben und Anteilsangaben, insbesondere solche zur Abgrenzung der Erfindung, soweit sie nicht die konkreten Beispiele betreffen, sind mit ± 10 % Toleranz zu verstehen, somit beispielsweise: 11% bedeutet: von 9,9% bis 12,1%. Prozentuelle Angaben von Bestandteilen sind, wenn nichts anderes angegeben ist, Gewichtsprozente. Bei Bezeichnungen wie bei: "ein Lösungsmittel" ist das Wort "ein" nicht als Zahlwort, sondern als Fürwort bzw. als unbestimmter Artikel anzusehen, wenn nicht aus dem Zusammenhang etwas anderes hervorgeht.

Der Begriff: "Kombination" bzw. "Kombinationen" steht, sofern nichts anderes angegeben, für alle Arten von Kombinationen, ausgehend von zwei der betreffenden Bestandteile bis zu einer Vielzahl derartiger Bestandteile bis zu allen Bestandteilen, der Begriff: "enthaltend" steht auch für "bestehend aus".

### REFERENZEN:

### Nicht-Patent-Literatur (gekürzt auf die wesentlichsten Publikationen)

Babourina O, Rengel Z. Uptake of aluminium into Arabidopsis root cells measured by fluorescent lifetime imaging. Ann Bot. 2009; 104(1): 189-195. doi: 10.1093/aob/mcp098
Caschetto S, Wollast R. Dissolved aluminum in interstitial waters of recent marine sediments. Geochim Cosmochim Acta. 1979; 43: 425-428.
Coons AH, Creech HJ, Jones RN. Immunological properties of an antibody containing a fluorescent group. Proc Soc Exp Biol Med. 1941; 47: 200-202.
Coons AH, Creech HJ, Jones RN, Berliner E. The demonstration of pneumococcal antigen in tissues by the use of fluorescent antibody. J Immunol. 1942; 45: 159-170.
Coons AH, Melvin H, Kaplan MH. Localization of antigen in tissue cells: II. Improvements in a method for the detection of antigen by means of fluorescent antibody. J Exp Med. 1950; 91(1): 1-13.
Cordes T, Maiser A, Steinhauer C, Schermelleh L, Tinnefeld P. Mechanisms and advancement of antifading agents for fluorescence microscopy and single-molecule spectroscopy. Phys Chem Chem Phys. 2011; 13(14): 6699-6709. doi: 10.1039/c0cp01919d
Crompton TR. Determination of metals in natural waters, sediments, and soils. Elsevier, Amsterdam. 2015; 1st Edition: 318 Seiten.
Eticha D, Stass A, Horst WJ. Localization of aluminium in the maize root apex: can morin detect cell wall-bound aluminium? J Exp Bot. 2005; 56(415): 1351-1357. doi:10.1093/jxb/eri136
Florijn RJ, Slats J, Tanke HJ, Raap AK. Analysis of antifading reagents for fluorescence microscopy. Cytometry. 1995; 19(2): 177-182. doi: 10.1002/cyto.990190213
He H-B, Lee H-K, Li SFY, Hsieh A-K, Chi H, Siow K-S. Determination of Trace Levels of Aluminum by Capillary Electrophoresis with Lumogallion Fluorometric Detection. J Chromatogr Sci. 1997; 35(7): 333-336. doi: 10.1093/chromsci/35.7.333
HuffJC, Weston WL, Wanda KD. Enhancement of specific immunofluorescent findings with use of a para-phenylenediamine mounting buffer. J Invest Dermatol. 1982; 78(5): 449-450.
Hydes DJ, Liss PS. The determination of low concentrations of dissolved aluminum in natural waters. Analyst. 1976; 101: 922-931.
Illés P, Schlicht M, Pavlovkin J, Lichtscheidl I, Baluska F, Ovecka M. Aluminium toxicity in plants: internalization of aluminium into cells of the transition zone in Arabidopsis root apices related to changes in plasma membrane potential, endosomal behaviour, and nitric oxide production. J Exp Bot. 2006; 57(15): 4201-4213. doi:10.1093/jxb/erl197
Johnson GD, Nogueira Araujo GM. A simple method of reducing the fading of immunofluorescence during microscopy. J Immunol Methods. 1981; 43: 349-350.
Johnson GD, Davidson RS, McNamee KC, Russell G, Goodwin D, Holborow EJ. Fading of immunofluorescence during microscopy: a study of the phenomenon and its remedy. J Immunol Methods. 1982; 55(2): 231-242.
Jonasson RG. Investigation of the reactions of aquatic aluminum through fluorometry using lumogallion. McMaster University, 1980. [http://hdl.handle.net/11375/18023]. A Thesis Submitted to the Department of Geology in Partial Fulfilment of the Requiments for the Degree Bachelor of Science.
Kataoka T, Iikura H, Nakanishi TM. Aluminum distribution and viability of plant root and cultured cells. Soil Science and Plant Nutrition. 1997; 43: 1003-1007. doi: 10.1080/00380768.1997.11863707
Klein JP, Mold M, Mery L, Cottier M, Exley C. Aluminum content of human semen: implications for semen quality. Reprod Toxicol. 2014; 50: 43-48. doi: 10.1016/j.reprotox.2014.10.001
Lee BL, Chua LH, Ong HY, Yang HG, Wu J, Ong CN. Determination of serum and urinary aluminum by HPLC with fluorometric detection of Allumogallion complex. Clin Chem. 1996; 42(9): 1405-1411.
Li Z, Xing F, Xing D. Characterization of target site of aluminum phytotoxicity in photosynthetic electron transport by fluorescence techniques in tobacco leaves. Plant Cell Physiol. 2012; 53(7): 1295-1309. doi: 10.1093/pcp/pcs076
Longin A, Souchier C, Ffrench M, Bryon PA. Comparison of anti-fading agents used in fluorescence microscopy: image analysis and laser confocal microscopy study. J Histochem Cytochem. 1993; 41(12): 1833-1840.
Mile I, Svensson A, Darabi A, Mold M, Siesjö P, Eriksson H. Al adjuvants can be tracked in viable cells by lumogallion staining. J Immunol Methods. 2015; 422: 87-94. doi: 10.1016/j.jim.2015.04.008
Ming DW, Dixon JB. Quantitative Determination of Clinoptilolite in Soils by a Cation-Exchange Capacity Method. Clays and Clay Minerals. 1987; 35: 463-468.
Mirza A, King A, Troakes C, Exley C. The Identification of Aluminum in Human Brain Tissue Using Lumogallion and Fluorescence Microscopy. J Alzheimers Dis. 2016; 54(4): 1333-1338. doi: 10.3233/JAD-160648
Mirza A, King A, Troakes C, Exley C. Aluminium in brain tissue in familial Alzheimer's disease. J Trace Elem Med Biol. 2017; 40: 30-36. doi: 10.1016/j.jtemb.2016.12.001
Mold M, Eriksson H, Siesjö P, Darabi A, Shardlow E, Exley C. Unequivocal identification of intracellular aluminium adjuvant in a monocytic THP-1 cell line. Sci Rep. 2014; 4: 6287. doi: 10.1038/srep06287
Mold M, Shardlow E, Exley C. Insight into the cellular fate and toxicity of aluminium adjuvants used in clinically approved human vaccinations. Sci Rep. 2016; 6: 31578. doi: 10.1038/srep31578
Mumpton FA. La roca magica: uses of natural zeolites in agriculture and industry. Proc Natl Acad Sci USA. 1999; 96(7): 3463-3470.
Nishikawa Y, Hiraki K, Morishige, Shigematsu T. Fluorophotometric determination of aluminum and gallium with lumogallion. Japan Analyst (Bunseki Kagaku). 1967; 16: 692-697.
Nishikawa Y, Hiraki K, Morishige K, Tsuchiyama A, Shigematsu T. Fluorometric determination of trace amount of aluminum in sea water. Japan Analyst (Bunseki Kagaku). 1968; 17: 1092-1097.
Platt JL, Michael AF. Retardation of fading and enhancement of intensity of immunofluorescence by p-phenylenediamine. J Histochem Cytochem. 1983; 31(6): 840-842. doi: 10.1177/31.6.6341464
Powell JJ. Analysis of aluminosilicate particles in biological matrices using histochemistry and X-ray microanalysis. Analyst. 2002; 127: 842-846. doi: 10.1039/B110761P
Ramos Jareño ME. Dissolution mechanism of montmorillonite in synthetic lung fluids: effect of organic ligands and biodurability. Doctoral dissertation. Granada: Universidad de Granada, 2013. 294 p. Retrieved from http://digibug.ugr.es/handle/10481/29552#.WYLoR7mQqHs.
Ren JL, Zhang J, Luo JQ, Pei XK, Jiang ZX. Improved fluorimetric determination of dissolved aluminium by micelle-enhanced lumogallion complex in natural waters. Analyst. 2001; 126(5): 698-702. doi: 10.1039/b007593k
Shigematsu T, Nishikawa Y, Hiraki K, Nagano N. Fluorometric determination of trace amount of aluminum in natural water by lumogallion method. Masking of ferric iron with o-phenanthroline. Japan Analyst (Bunseki Kagaku). 1970; 19(4): 551-554.
Sivaguru M, Liu J, Kochian LV. Targeted expression of SbMATE in the root distal transition zone is responsible for sorghum aluminum resistance. Plant J. 2013; 76(2): 297-307. doi: 10.1111/tpj.12290
Sposito G. The environmental chemistry of aluminum. CRC Press, Boca Raton. 1995; 2nd Edition: 480 Seiten.
Sutheimer SH, Maurice PA, Zhou Q. Dissolution of well and poorly crystallized kaolinites; Al speciation and effects of surface characteristics. American Mineralogist. 1999; 84(4): 620-628. doi: 10.2138/am-1999-0415
Tibbett M, Carter DO. Soil Analysis in Forensic Taphonomy: Chemical and Biological Effects of Buried Human Remains. CRC Press. 27.02.2008; 364 Seiten.
Uchiumi A, Takatsu A, Teraki Y. Sensitive detection of trace aluminium in biological tissues by confocal laser scanning microscopy after staining with lumogallion. Analyst. 1998; 123(4): 759-762.
Valnes K, Brandtzaeg P. Retardation of immunofluorescence fading during microscopy. J Histochem Cytochem. 1985; 33(8): 755-761. doi:10.1177/33.8.3926864
Yamada E, Hiwada T, Inaba T, Tokukura M, Fuse Y. Speciation of aluminum in soil extracts using cation and anion exchangers followed by a flow-injection system with fluorescence detection using lumogallion. Anal Sci. 2002; 18(7): 785-791.
Zhou Y, Yokel RA. The chemical species of aluminum influences its paracellular flux across and uptake into Caco-2 cells, a model of gastrointestinal absorption. Toxicol Sci. 2005; 87(1): 15-26. doi: 10.1093/toxsci/kfi216

### Patent-Literatur:

- Anti-counterfeit penetrant atomic stamp pad ink and its preparation method, 1996 CN 96104762
- Anti-fake fluorescent pressure-sensitive carbon paper generating invisible duplication handwriting and making method thereof, 1996 CN 96100340
- Anti-fake fluorescent pressure-sensitive carbon paper, 1996 CN 96100339
- Changed condition indicator, 2001 US 11/375,307
- Chelate reagent and measuring aluminum and measuring method, 2000 US 10/380,242
- Detector used for investigating water consists of an active ingredient coating formed on a carrier, 2000 DE2000160845
- Diagnostic test for elemental imbalances, 2002 PCT/US2003/012911 und EP20030719936
- Dye lasers, 1978 US 05/963,157
- Fiber optic moisture sensor, 1992 US 07/914,795 und US 08/201,820
- Fluorescent anti-fake mark and its making method, 1994 CN 94105055
- Fluorescent probe for the detection of corrosion, 2005 PCT/GB2006/001626
- Method for analyzing trace quantity of Aluminum, 2003 JP20020104985 20020408
- Method for determination of aluminum, 2001 US 10/498,060
- Methods of monitoring rinsing solutions and related systems and reagents, 2004 US 10/952,510
- Method of producing crosslinkable silyl group-containing polyoxyalkylene polymers, 2000 US 09/832,344 und EP20010109302
- Organic electroluminescence device, 2000
   US 09/915,452
- Organic LED element, 1999 JP19980083987 19980330
- pH-sensitive microparticles with matrix-dispersed active agent, 2011 US 13/542,155
- Photodeposition method for fabricating a three-dimensional, patterned polymer microstructure, 1995 US 08/519,062
- Semiconductor device and adhesive sheet, 2009 CN 201010135730
- Semiconductor wafer cleaning agent and cleaning method, 2000 US 10/203,659
- Synergistic combinations of chromate-free corrosion inhibitors, 2005 PCT/US2006/007305 und US 11/817,659
- Verfahren zur Herstellung vernetzbarer Silylgruppen enthaltender Polyoxyalkylenpolymere, 2000 DE2001625268

## Patentansprüche

1. Verfahren zur spezifischen Fluoreszenz-Markierung von Alumosilikaten, im Speziellen von Zeolithen und insbesondere von von Schwermetallen aufgereinigten Klinoptilolithen, mit Lumogallion (5-Chloro-3-(2,4-dihydroxyphenylazo)-2-hydroxybenzenesulfonsäure), wobei die Alumosilikate mit einer Lumogallion-Lösung in einem Pufferbereich von physiologisch bei pH 7,2 bis sauer bei pH 4,0 entweder für kurze Zeit von 2 bis 4 Stunden bei hohen Temperaturen von 60 bis 80 °C oder für längere Zeit von 12 bis 24 Stunden bei niedrigen Temperaturen von 20 bis 30 °C inkubiert und mithilfe eines Eindeckmediums stabilisiert werden, **dadurch gekennzeichnet, dass** das Eindeckmedium para-Phenylendiamin (1,4-Diaminobenzol) in Glycerin enthält bzw. daraus besteht.

2. Verfahren nach Anspruch 1, **dadurch gekennzeichnet, dass** es in zellbiologischen und histologischen Proben, welche entweder in Paraffin oder Paraffin-Ersatz eingebettet worden waren, durchgeführt wird

3. Verfahren nach Anspruch 1 und 2 **dadurch gekennzeichnet, dass** die in Paraffin oder Paraffin-Ersatz eingebetteten Proben aufgrund des Verfahrens auch ohne Entparaffinierung/Entwachsung markiert werden können.

4. Verfahren nach Anspruch 1, **dadurch gekennzeichnet, dass** es in mineralogischen und geologischen Proben, welche entweder in Paraffin oder Paraffin-Ersatz eingebettet worden waren, durchgeführt wird

5. Verfahren nach Anspruch 1, **dadurch gekennzeichnet, dass** es in In-vitro-Zellkultur-Proben, welche mit Alumosilikaten oder Gemischen aus Alumosilikaten und Nicht-Alumosilikaten inkubiert und anschließend fixiert worden waren, durchgeführt wird.

6. Verfahren nach einem der Ansprüche 1 bis 4, **dadurch gekennzeichnet, dass** es im Rahmen eines analytisch-diagnostischen Schnellnachweises oder zur bildlichen Darstellung eingesetzt wird.

## Claims

1. A method for the specific fluorescence labeling of aluminosilicates, in particular of zeolites, and in particular of clinoptilolites purified from heavy metals, with lumogallion (5-chloro-3-(2,4-dihydroxyphenylazo)-2-hydroxybenzenesulfonic acid), whereby the aluminosilicates are incubated with a lumogallion solution in a buffer ranging from physiological pH 7.2 to acidic pH 4.0, either for a short period of time from 2 to 4 hours at high temperatures of 60 to 80 °C, or for a longer period of time from 12 to 24 hours at low temperatures of 20 to 30 °C, and stabilized using a mounting medium, **characterized in that**, the mounting medium contains or consists of para-phenylenediamine (1,4-diaminobenzene) in glycerol.

2. The method according to Claim 1, **characterized in that** it is carried out in cell biological and histological samples, which were embedded in either paraffin or paraffin substitute.

3. The method according to Claim 1 and 2, **characterized in that**, due to the method, the samples embedded in paraffin or paraffin substitutes can be labeled without dewaxing.

4. The method according to Claim 1, **characterized in that** it is carried out in mineralogical and geological samples, which were embedded in either paraffin or paraffin substitute.

5. The method according to Claim 1, **characterized in that** it is carried out in in vitro-cell culture samples which are incubated with aluminosilicates or mixtures of aluminosilicates and non-aluminosilicates, then fixed.

6. The method according to any of Claims 1 to 4, **characterized in that** it is used in the context of an analytical-diagnostic rapid detection or for imaging.

## Revendications

1. Procédé de marquage fluorescent spécifique d'aluminosilicates, en particulier de zéolithes et notamment de clinoptilolithes débarrassées des métaux lourds, avec du lumogallion (5-chloro-3-(2,4-dihydroxyphenylazo)-2-hydroxybenzène-acide sulfonique), selon lequel les aluminosilicates sont incubés avec une solution de lumogallion, dans un domaine tampon allant d'un pH physiologique de 7,2 à un pH acide de 4,0, soit pour une courte durée allant de 2 à 4 heures, à des températures élevées, comprises entre 60 et 80 C, soit pour des durées plus longues, allant de 12 à 24 heures, à des températures faibles, comprises entre 20 et 30 C, et sont stabilisés à l'aide d'un milieu de montage, **caractérisé en ce que** le milieu de montage contient de la paraphénylènediamine (1,4-diaminobenzène) dans de la glycérine ou est constitué de celle-ci.

2. Procédé selon la revendication 1, **caractérisé en ce qu'**il est mis en œuvre dans des échantillons biologiques cellulaires et histologiques qui étaient inclus soit en paraffine soit dans un substitut de paraffine.

3. Procédé selon les revendications 1 et 2, **caractérisé en ce que** grâce au procédé, les échantillons inclus en paraffine ou dans un substitut de paraffine peuvent être marqués également sans déparaffinage/décirage.

4. Procédé selon la revendication 1, **caractérisé en ce qu'**il est mis en œuvre dans des échantillons minéralogiques et géologiques qui étaient inclus soit en paraffine soit dans un substitut de paraffine.

5. Procédé selon la revendication 1, **caractérisé en ce qu'**il est mis en œuvre dans des échantillons de culture cellulaire in vitro qui étaient incubés avec des aluminosilicates ou des mélanges d'aluminosilicates et de non aluminosilicates et étaient ensuite fixés.

6. Procédé selon l'une des revendications 1 à 4, **caractérisé en ce qu'**il est utilisé dans le cadre d'une détection analytique diagnostique rapide ou à des fins de représentation graphique.
